# EUROPEAN PATENT APPLICATION

(11) **EP 4 477 661 A1**
(43) Date of publication of application: **18.12.2024**
(21) Application number: 22925709.2
(22) Date of filing: 02.12.2022
(51) Int. Cl.: C07K 14/00, A61P 31/20, A61P 31/14, A61P 1/16, A61K 38/16

(54) **LONG-LASTING HEPATITIS VIRUS ENTRY INHIBITOR**

(30) Priority: 10.02.2022 CN 202210126691
(71) Applicant: Chengdu Aoda Biotechnology Co., Ltd., Chengdu, Sichuan 610041 (CN)
(72) Inventor: YU, Haining, Chengdu, Sichuan 610041 (CN); WANG, Peng, Chengdu, Sichuan 610041 (CN); ZHOU, Shuliang, Chengdu, Sichuan 610041 (CN); GAO, Junping, Chengdu, Sichuan 610041 (CN)
(74) Representative: PGA S.p.A., Milano, Succursale di Lugano
(86) International application number: PCT/CN2022/136263
(87) International publication number: WO 2023/151358

(57) **Abstract**

The present invention relates to the field of pharmaceutical synthesis. Disclosed is a long-lasting hepatitis virus entry inhibitor. The long-lasting hepatitis virus entry inhibitor of the present invention is used for preparing a pharmaceutical composition for treating diseases, the pharmaceutical composition being used for treating chronic hepatitis B and hepatitis D.

## Description

This application claims the priority of Chinese Patent Application No. 202210126691.4, filed with the China National Intellectual Property Administration on February 10, 2022, and titled with "LONG-LASTING HEPATITIS VIRUS ENTRY INHIBITOR", the disclosure of which is hereby incorporated by reference in its entirety.

### FIELD

The present disclosure relates to a long-lasting hepatitis virus entry inhibitor and use thereof.

### BACKGROUND

Viral hepatitis is an infectious disease caused by a variety of hepatitis viruses, mainly involving liver lesions. The main clinical manifestations are loss of appetite, nausea, epigastric discomfort, pain in the hepatic region, and fatigue. Some patients may have jaundice with fever and liver function impairment. Some patients may have chronic hepatitis, or even develop into cirrhosis, and a few may develop into liver cancer.

The hepatitis viruses involved in viral hepatitis include five hepatitis viruses including hepatitis A, B, C, D, and E viruses according to etiological typing, namely hepatitis A virus (HAV), hepatitis B virus (HBV), hepatitis C virus (HCV), hepatitis D virus (HDV) and hepatitis E virus (HEV), respectively. Except for hepatitis B virus which is a DNA virus, the others are RNA viruses.

HAV belongs to the genus of hepatovirus in the family of Picornaviridae. Most human infections with HAV manifest as subclinical or occult infections, and only a few manifest as acute hepatitis A. Generally, complete recovery is possible without turning into chronic hepatitis, and there are no chronic carriers.

HBV is a pathogen causing hepatitis B, belonging to the family of hepadnaviridae including two genera of orthohepadnavirus and avihepadnavirus, in which genus orthohepadnavirus can cause infection in human beings. HBV infection is a global public health problem, with the production and investment of genetically engineered vaccines increases, the popularity of HBV vaccine has increased year by year, and the infection rate has shown a decreasing trend. There are about 2 billion HBV-infected patients worldwide, of which 380 million infected patients manifest as chronic infection, wherein the virus harbor in tissues and organs, and thus cannot be cleared by the immune system or drugs.

HCV is mainly transmitted through blood, sexual intercourse, mother-to-child transmission (vertical transmission). However, it is a great pity that no effective vaccine against HCV is developed in medical science yet. Because the hepatitis C virus is an RNA virus that is highly mutable, and no other animals except humans and chimpanzees can be with infected hepatitis C virus, it is thus difficult to develop a vaccine resulting from the difficulty in finding an animal model. But a new generation of oral direct-acting antiviral drug has made it practicable to cure hepatitis C.

HDV is a defective, single-stranded, negative sense RNA virus that depends on hepatophilic DNA viruses, such as HBV, to provide its capsid for replication. HDV is found in the nuclei of hepatocytes and in serum of HBsAg-positive HDV-infected patients. The replication of HDV is mainly performed in hepatocytes. HDV is prone to mutation. After infection with HDV in human, HBV-DNA synthesis can be significantly inhibited, and the occurrence of HDAg coincides with a decrease in serum HBV-DNA, and the HBV-DNA returns to its initial level with HDAg turned negative and the occurrence of anti-HD. HDV is mainly transmitted by blood transfusion and blood products, similar to the mode of transmission of the hepatitis B virus. HDV infection is most commonly found in HBV-infected patients, but sporadic HDV infection may also occur. Overlapping infection with HDV and HBV can aggravate liver damage, which may be prone to progress into chronic active hepatitis, cirrhosis and severe hepatitis.

Researchers have found that a protein on the surface of liver cells called hepatic bile acid transporter (sodium taurocholate cotransporter polypeptide, NTCP) is able to interact specifically with the key receptor-binding domains of the HBV envelope proteins, which is the receptor required for HBV to infect host cells. Therefore, blocking NTCP may hopefully cure hepatitis B.

Bulevirtide is a 47 amino acid drug targeting NTCP, which has a short half-life in the body and requires patients to take medication every day for a long term, resulting in poor compliance of patients and high clinical costs. The present disclosure aims to provide a long-lasting hepatitis virus entry inhibitor for patients to reduce the frequency of drug administration and costs.

### SUMMARY

The present disclosure provides a long-lasting hepatitis virus entry inhibitor and use thereof.

In order to achieve the above object, the present disclosure first provides a compound represented by Formula I, a pharmaceutically acceptable salt thereof, a solvate thereof, a chelate thereof, a non-covalent complex thereof, a prodrug thereof, or any mixture thereof. 
R-Gly-Thr-Asn-Leu-Ser-Val-Pro-Asn-Pro-Leu-Gly-Phe-Phe-Pro-Asp-His-Gln-Leu-Asp-Pro-Ala-
Phe-Gly-Ala-Asn-Ser-Asn-Asn-Pro-Asp-Trp-Asp-Phe-Asn-Pro-Asn-Lys-Asp-His-Trp-Pro-Glu-A
la-Asn-AA1-Val-Gly-AA2
Formula I
wherein AA1 of Formula I is selected from the group consisting of Arg, Lys, Gln, Glu, and Cit;
AA2 of Formula I is NH₂ or OH; and
R of Formula I is HO₂C(CH₂)ₙ₁CO-(AA3)ₙ₂-(PEGₙ₃(CH₂)ₙ₄CO)ₙ₅-, or HO₂C(CH₂)ₙ₁CO-(AA3)ₙ₂-(AA4)ₙ₆-;
wherein, n1 is an integer selected from 10 to 20,
n2 is an integer selected from 1 to 5,
n3 is an integer selected from 1 to 30,
n4 is an integer selected from 1 to 5,
n5 is 0, or is an integer selected from 1 to 5, and
n6 is 0, or is an integer selected from 1 to 10;
AA3 is selected from the group consisting of yGlu, εLys, β-Ala, γ-aminobutyric acid, and 5-Ava; and
AA4 is selected from the group consisting of Ala, Gly, Leu, Ser, Thr, Tyr, Gln, Lys, Dao, Dah, Orn, Dab, Dap, Glu, Asp, Ada, Apm, and Asu.

If n6 is an integer selected from 2 to 10, the repeating unit of AA4 can be the same amino acid or different amino acids.

In some embodiments, AA1 is selected from the group consisting of Arg, Lys, and Gln, and AA2 is NH₂.

In some embodiments, R is eicosanedioic acid-γGlu-(AA4)ₙ₆-, AA4 is selected from the group consisting of Gly, Ser, Thr, Asn, Leu, Val, and Pro; and n6 is 0 or an integer selected from 1 to 7. In some other embodiments, R is eicosanedioic acid-γGlu-PEGₙ₃-CH₂CO-; wherein n3 is an integer selected from 1 to 6.

In some embodiments, R is selected from the group consisting of eicosanedioic acid-yGlu-,
eicosanedioic acid-γGlu-PEG₁CH₂CO-,
eicosanedioic acid-γGlu-PEG₂CH₂CO-,
eicosanedioic acid-γGlu-PEG₃CH₂CO-,
eicosanedioic acid-γGlu-PEG₄CH₂CO-,
eicosanedioic acid-γGlu-PEG₅CH₂CO-,
eicosanedioic acid-γGlu-PEG₆CH₂CO-,
eicosanedioic acid-yGlu-Gly-,
eicosanedioic acid-yGlu-Gly-Gly-,
eicosanedioic acid-yGlu-Gly-Gly-Gly-,
eicosanedioic acid-yGlu-Gly-Gly-Gly-Gly-,
eicosanedioic acid-yGlu-Gly-Gly-Gly-Gly-Gly-,
eicosanedioic acid-yGlu-Gly-Gly-Gly-Gly-Gly-Gly-Gly-,
eicosanedioic acid-yGlu-Gly-Gly-Ser-Gly-Ser-Gly-, and
eicosanedioic acid-yGlu-Gly- Thr-Asn-Leu-Ser- Val-Pro-Asn-Pro-Leu-.

The long-acting hepatitis virus entry inhibitor of the present disclosure comprises the compound, and the compound is in a form selected from the group consisting of a pharmaceutically acceptable salt thereof, a solvate thereof, a chelate thereof, a non-covalent complex thereof, a prodrug thereof, and a mixture thereof.

The present disclosure further provides a pharmaceutical composition comprising the compound according to the present disclosure, as well as use of the pharmaceutical composition of the present disclosure in the manufacture of a medicament for treating a disease.

Further, the pharmaceutical compositions can be used in the treatment of a chronic disease caused by hepatitis B and D.

More contents of the present disclosure are described in detail below, or some of them may be illustrated in the embodiments of the present disclosure.

Unless otherwise indicated, the amounts of various components and the reaction conditions used herein may be interpreted as "roughly" or "approximately" in any case. Correspondingly, unless otherwise specified, the numerical parameters quoted herein and in the claims are approximate parameters, and different numerical parameters may be obtained due to differences in standard errors under respective experimental conditions.

When there is disagreement or doubt between the chemical structural formula and the chemical name of a compound herein, the chemical structural formula is used to exactly define the compound. The compound described herein may contain one or more chiral centers, and/or double bonds and the like, and may also exist as stereoisomers, including double bond isomers (such as geometric isomers), optically active enantiomers or diastereomers. Accordingly, any chemical structure within the scope of the description herein, whether part of or the whole structure containing similar structures above, includes all possible enantiomers and diastereomers of the compound, including any of the pure stereoisomers (such as pure geometric isomers, pure enantiomers or pure diastereomers) and any mixture of these isomers. These racemic and stereoisomeric mixtures can also be further resolved into their constituent enantiomers or stereoisomers by those skilled in the art using different separation techniques or chiral molecular synthesis methods.

The compound represented by Formula I includes, but is not limited to, optical isomers, racemates and/or other mixtures thereof. In the above case, pure enantiomer or diastereomer, such as optical isomer, can be obtained by asymmetric synthesis or resolution of racemates. Resolution of racemates can be accomplished by various methods, such as conventional recrystallization with a resolution-promoting agent, or by chromatography. In addition, the compound represented by Formula I also includes cis- and/or trans-isomers with double bonds.

The compound of the present disclosure includes, but is not limited to, the compound represented by Formula I and all various pharmaceutically acceptable forms. The various pharmaceutically acceptable forms include various pharmaceutically acceptable salts, solvates, complexes, chelates, non-covalent complexes, prodrugs based on the aforementioned substances and any mixture of the aforementioned forms.

### DETAILED DESCRIPTION

The present disclosure discloses a long-lasting hepatitis virus entry inhibitor and use thereof. Those skilled in the art can learn from the content of the present disclosure and appropriately improve relevant parameters for implementation. It should be particularly noted that all similar substitutions and modifications are obvious to those skilled in the art, and they are deemed to be included in the present disclosure. The method of the present disclosure has been described through the preferred embodiments, and it is obvious that those skilled can achieve and apply the techniques of the present disclosure by changes or appropriate modifications and combinations of the compound and the preparation method described herein, without departing from the content, spirit and scope of the preset disclosure.

The terms corresponding to the abbreviations used in the present disclosure are shown in the following table:

| Abbreviation | Term | Abbreviation | Term |
|---|---|---|---|
| Fmoc | 9-Fluorenemethoxycarbonyl | OtBu | Tert-butoxy |
| tBu | Tert-butyl | Boc | Tert-butoxycarbonyl |
| Trt | Trityl | Pbf | (2,3-Dihydro-2,2,4,6,7-pentameth ylbenzofuran-5-yl)sulfonyl |
| Ala | Alanine | Leu | Leucine |
| Arg | Arginine | Lys | Lysine |
| Asn | Asparagine | Met | Methionine |
| Asp | Aspartic acid | Phe | Phenylalanine |
| Cys | Cysteine | Pro | Proline |
| Gln | Glutamine | Ser | Serine |
| Glu | Glutamic acid | Thr | Threonine |
| Gly | Glycine | Trp | Tryptophan |
| His | Histidine | Tyr | Tyrosine |
| Ile | Isoleucine | Val | Valine |
| Aib | Aminoisobutyric acid | Dah | 2,7-diaminoheptanoic acid |
| 5-Ava | 5-Aminovaleric acid | Dao | 2,8-diaminooctanoic acid |
| Dap | 2,3-Diaminopropionic acid | Ada | 2-aminoadipic acid |
| Dab | 2,4-Diaminobutyric acid | Apm | 2-aminopimelic acid |
| Orn | Ornithine | Asu | 2-aminooctanedioic acid |

### Example 1 Preparation of compound

The preparation method comprises: preparing a peptide resin by a solid-phase polypeptide synthesis method, then subjecting the peptide resin to acid hydrolysis to obtain a crude product, and finally purifying the crude product to obtain a pure product; wherein the step of preparing a peptide resin by a solid-phase polypeptide synthesis method is performed by sequentially coupling the protected amino acid or fragment corresponding to the following sequences to a carrier resin by solid-phase coupling synthesis to prepare the peptide resin.

In the above preparation method, the amount of the Fmoc-protected amino acid or the protected amino acid fragment is 1.2-6 times, preferably 2.5-3.5 times of the total moles of the used resin.

In the above preparation method, the degree of substitution of the carrier resin is 0.2-1.0 mmol/g of resin, preferably 0.3-0.5 mmol/g of resin.

As a preferred embodiment of the present disclosure, the solid-phase coupling synthesis method comprises: removing the Fmoc protecting group from the protected amino acid-resin obtained in the previous step, and subjecting the resulting resin to a coupling reaction with the next protected amino acid. The removal of the Fmoc protecting group is performed for 10-60 min, preferably for 15-25 min. The coupling reaction is performed for 60-300 min, preferably for 100-140 min.

The coupling reaction requires the addition of a condensing agent, and the condensing agent is selected from the group consisting of DIC (N,N-diisopropylcarbodiimide), N,N-dicyclohexylcarbodiimide, benzotriazole-1-yl-oxytripyrrolidinophosphonium hexafluorophosphate, 2-(7-aza-1H-benzotriazole-1-yl)-1,1,3,3-tetramethyluronium hexafluorophosphate, benzotriazole-N,N,N',N'-tetramethyluronium hexafluorophosphate and O-benzotriazole-N,N,N',N'-tetramethyluronium tetrafluoroborate; preferably N,N-diisopropylcarbodiimide. The molar amount of the condensing agent is 1.2-6 times, preferably 2.5-3.5 times, of the total moles of amino groups in the amino resin.

The coupling reaction requires the addition of an activating agent, and the activating agent is selected from the group consisting of 1-hydroxybenzotriazole and N-hydroxy-7-azabenzotriazole, preferably 1-hydroxybenzotriazole. The amount of the activating agent is 1.2-6 times, preferably 2.5-3.5 times, of the total moles of amino groups in the amino resin.

As a preferred embodiment of the present disclosure, a reagent for removing Fmoc protection is a mixed solution of PIP/DMF (piperidine/N,N-dimethylformamide), and the content of piperidine in the mixed solution is 10-30% (V). The amount of the reagent for removing Fmoc protection is 5-15 mL per gram of amino resin, preferably 8-12 mL per gram of amino resin.

Preferably, the peptide resin is subjected to acid hydrolysis to remove both the resin and the protecting group of side chain to obtain a crude product.

Further preferably, an acid hydrolyzing agent used during acid hydrolysis of the peptide resin is a mixed solvent of trifluoroacetic acid (TFA), 1,2-ethanedithiol (EDT) and water, and the volume percentage of each component in the mixed solvent is as follows: TFA 80-95%, EDT 1-10%, and the balance is water.

More preferably, the volume percentage of each component in the mixed solvent is as follows: TFA 89-91%, EDT 4-6%, and the balance is water. The optimal volume percentage of each component in the mixed solvent is as follows: TFA 90%, EDT 5%, and the balance is water.

The amount of the acid hydrolyzing agent used is 4-15 mL of acid hydrolyzing agent per gram of peptide resin; preferably, 7-10 mL of acid hydrolyzing agent per gram of peptide resin.

The acid hydrolysis using the acid hydrolyzing agent is performed for 1-6 hours at room temperature, preferably for 3 to 4 hours.

Further, the crude product is purified by high performance liquid chromatography and freeze-dried to obtain a pure product.

### 1. Synthesis of peptide resin

Using Rink Amide BHHA resin as the carrier resin, the corresponding protected amino acids were sequentially coupled by removing Fmoc protection and performing coupling reaction.

### (1) Coupling a first protected amino acid in main chain

0.03 mol of the first protected amino acid and 0.03 mol of HOBt were dissolved in an appropriate amount of DMF. 0.03 mol of DIC was then slowly added to the DMF solution of the protected amino acid under stirring, stirred and reacted at room temperature for 30 min to obtain an activated solution of the protected amino acid for later use.

0.01 mol of Rink amide MBHA resin (degree of substitution was about 0.4 mmol/g) was subjected to deprotection with 20% PIP/DMF solution for 25 min, washed and filtered to obtain a Fmoc-removed resin.

The activated solution of the first protected amino acid was added to the Fmoc-removed resin for coupling reaction for 60-300 min, filtered and washed to obtain a resin containing the first protected amino acid.

### (2) Coupling the 2nd to 49th protected amino acids in main chain

The above-mentioned corresponding 2nd to 49st protected amino acids were sequentially coupled by the same method as above for coupling the first protected amino acid in the main chain to obtain a resin containing 49 amino acids in the main chain.

### (3) Coupling a first protected amino acid in side chain

0.03 mol of a first protected amino acid in side chain and 0.03 mol of HOBt were dissolved in an appropriate amount of DMF. 0.03 mol of DIC was slowly added to the DMF solution of the protected amino acid under stirring, stirred and reacted at room temperature for 30 min to obtain the activated solution of the protected amino acid.

2.5 mmol of tetrakistriphenylphosphine palladium and 25 mmol of phenylsilane were dissolved in an appropriate amount of dichloromethane, subjected to deprotection for 4 hours, filtered and washed to obtain Alloc-removed resin for later use.

The activated solution of the first protected amino acid in side chain was added to the Alloc-removed resin for coupling reaction for 60-300 min, filtered and washed to obtain a resin containing the first protected amino acid in side chain.

### (4) Coupling the 2nd to 4th protected amino acids in side chain

The 2nd to 4th protected amino acids corresponding to the side chain and monoprotected fatty acid were sequentially coupled by the same method as above for coupling the first protected amino acid in main chain to obtain a peptide resin.

### 2. Preparation of crude product

To the above peptide resin, a lysing agent with a volume ratio of TFA: water: EDT=95:5:5 (10 mL of lysing agent/g of resin) was added, stirred evenly and reacted under stirring at room temperature for 3 h. The reaction mixture was filtered with a sand core funnel, the filtrate was collected, and the resin was washed three times with a small amount of TFA. The filtrates were combined, concentrated under reduced pressure, and precipitated with anhydrous ether. The precipitate was washed with anhydrous ether three times, and dried by suction to obtain a crude product as an off-white powder.

### 3. Preparation of pure product

The above crude product was added with water and stirred. The pH of the solution was adjusted to 8.0 with aqueous ammonia until the crude product was completely dissolved. The solution was filtered with a 0.45 µm mixed microporous filtration membrane and purified for later use.

The purification was performed by high-performance liquid chromatography. The chromatographic packing material for purification was 10 µm reverse phase C18. The mobile phase system was 0.1%TFA/aqueous solution-0.1%TFA/acetonitrile solution. The flow rate of the 30 mm*250 mm chromatographic column was 20 mL/min. The elution was performed by a gradient system, and the loading was cycled for purification. The solution of the crude product was loaded into the chromatographic column and then eluted with the mobile phase. The eluate of the main peak was collected and subjected to evaporation to remove acetonitrile to obtain a purified intermediate concentrate.

The purified intermediate concentrate was filtered with a 0.45 µm filter membrane for later use. Salt exchange was performed by high-performance liquid chromatography. The mobile phase system was 1% acetic acid/aqueous solution-acetonitrile. The chromatographic packing material for purification was 10 µm reverse phase C18. The flow rate of the 30 mm*250 mm chromatographic column was 20 mL/min (the corresponding flow rate could be adjusted according to the chromatographic column of different specifications). The gradient elution and cyclic sample loading was performed. The sample was loaded into the chromatographic column and then eluted with the mobile phase. The eluates were collected and subjected to spectrum analysis, and change in absorbance was observed. The eluate of the main peak during salt exchange was collected and detected for purity using analytical liquid chromatography. The eluate of the main peak during salt exchange was combined, and concentrated under reduced pressure to obtain an aqueous acetic acid solution of pure product, which was then freeze-dried to obtain the pure product.

The following compounds were prepared by the above method:

| Number | Amino acid sequence |
|---|---|
| Compound 1 | eicosanedioic acid-γGlu-Gly-Thr-Asn-Leu-Ser-Val-Pro-Asn- Pro-Leu-Gly-Phe-Phe-Pro-Asp-His-Gln-Leu-Asp-Pro- Ala-Phe-Gly-Ala-Asn-Ser-Asn-Asn-Pro-Asp-Trp-Asp- Phe-Asn-Pro-Asn-Lys-Asp-His-Trp-Pro-Glu-Ala-Asn- Lys-Val-Gly-NH2 |
| Compound 2 | eicosanedioic acid-γGlu-PEG₁CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH₂ |
| Compound 3 | eicosanedioic acid-γGlu-PEG₂CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH₂ |
| Compound 4 | eicosanedioic acid-γGlu-PEG₃CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- |
| | Glu-Ala-Asn-Lys-Val-Gly-NH2 |
| Compound 5 | eicosanedioic acid-γGlu-PEG₄CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH₂ |
| Compound 6 | eicosanedioic acid-γGlu-PEG5CH2CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH2 |
| Compound 7 | eicosanedioic acid-γGlu-PEG₆CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH₂ |
| Compound 8 | eicosanedioic acid-γGlu-Gly-Gly-Thr-Asn-Leu-Ser-Val-Pro- Asn-Pro-Leu-Gly-Phe-Phe-Pro-Asp-His-Gln-Leu-Asp- Pro-Ala-Phe-Gly-Ala-Asn-Ser-Asn-Asn-Pro-Asp-Trp- Asp-Phe-Asn-Pro-Asn-Lys-Asp-His-Trp-Pro-Glu-Ala- Asn-Lys-Val-Gly-NH2 |
| Compound 9 | eicosanedioic acid-γGlu-Gly-Gly-Gly-Thr-Asn-Leu-Ser-Val- Pro-Asn-Pro-Leu-Gly-Phe-Phe-Pro-Asp-His-Gln-Leu- |
| | Asp-Pro-Ala-Phe-Gly-Ala-Asn-Ser-Asn-Asn-Pro-Asp- Trp-Asp-Phe-Asn-Pro-Asn-Lys-Asp-His-Trp-Pro-Glu- Ala-Asn-Lys-Val-Gly-NH2 |
| Compound 10 | eicosanedioic acid-γGlu-Gly-Gly-Gly-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Lys-Val-Gly-NH2 |
| Compound 11 | eicosanedioic acid-γGlu-Gly-Gly-Gly-Gly-Gly-Thr-Asn-Leu- Ser-Val-Pro-Asn-Pro-Leu-Gly-Phe-Phe-Pro-Asp-His- Gln-Leu-Asp-Pro-Ala-Phe-Gly-Ala-Asn-Ser-Asn-Asn- Pro-Asp-Trp-Asp-Phe-Asn-Pro-Asn-Lys-Asp-His-Trp- Pro-Glu-Ala-Asn-Lys-Val-Gly-NH₂ |
| Compound 12 | eicosanedioic acid-γGlu-Gly-Gly-Gly-Gly-Gly-Gly-Thr-Asn- Leu-Ser-Val-Pro-Asn-Pro-Leu-Gly-Phe-Phe-Pro-Asp- His-Gln-Leu-Asp-Pro-Ala-Phe-Gly-Ala-Asn-Ser-Asn- Asn-Pro-Asp-Trp-Asp-Phe-Asn-Pro-Asn-Lys-Asp-His- Trp-Pro-Glu-Ala-Asn-Lys-Val-Gly-NH2 |
| Compound 13 | eicosanedioic acid-γGlu-Gly-Gly-Gly-Gly-Gly-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- Asn-Asn-Pro-Asp-Trp-Asp-Phe-Asn-Pro-Asn-Lys-Asp- His-Trp-Pro-Glu-Ala-Asn-Lys-Val-Gly-NH2 |
| Compound 14 | eicosanedioic acid-γGlu-Gly-Gly-Ser-Gly-Ser-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- Asn-Asn-Pro-Asp-Trp-Asp-Phe-Asn-Pro-Asn-Lys-Asp- His-Trp-Pro-Glu-Ala-Asn-Lys-Val-Gly-NH2 |
| Compound 15 | eicosanedioic acid-γGlu-Gly-Thr-Asn-Leu-Ser-Val-Pro-Asn- Pro-Leu-Gly-Phe-Phe-Pro-Asp-His-Gln-Leu-Asp-Pro- Ala-Phe-Gly-Ala-Asn-Ser-Asn-Asn-Pro-Asp-Trp-Asp- Phe-Asn-Pro-Asn-Lys-Asp-His-Trp-Pro-Glu-Ala-Asn- Arg-Val-Gly-NH2 |
| Compound 16 | eicosanedioic acid-γGlu-PEG₁CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH₂ |
| Compound 17 | eicosanedioic acid-γGlu-PEG₂CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH₂ |
| Compound 18 | eicosanedioic acid-γGlu-PEG₃CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- |
| | Asp-Trp-Asp-Phe-Asn-Pro-Asn-Lys-Asp-His-Trp-Pro- Glu-Ala-Asn-Arg-Val-Gly-NH2 |
| Compound 19 | eicosanedioic acid-γGlu-PEG₄CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH₂ |
| Compound 20 | eicosanedioic acid-γGlu-PEG₅CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH₂ |
| Compound 21 | eicosanedioic acid-γGlu-PEG₆CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH₂ |
| Compound 22 | eicosanedioic acid-γGlu-Gly-Gly-Thr-Asn-Leu-Ser-Val-Pro- Asn-Pro-Leu-Gly-Phe-Phe-Pro-Asp-His-Gln-Leu-Asp- Pro-Ala-Phe-Gly-Ala-Asn-Ser-Asn-Asn-Pro-Asp-Trp- Asp-Phe-Asn-Pro-Asn-Lys-Asp-His-Trp-Pro-Glu-Ala- Asn-Arg-Val-Gly-NH2 |
| Compound 23 | eicosanedioic acid-γGlu-Gly-Gly-Gly-Thr-Asn-Leu-Ser-Val- |
| | Pro-Asn-Pro-Leu-Gly-Phe-Phe-Pro-Asp-His-Gln-Leu- Asp-Pro-Ala-Phe-Gly-Ala-Asn-Ser-Asn-Asn-Pro-Asp- Trp-Asp-Phe-Asn-Pro-Asn-Lys-Asp-His-Trp-Pro-Glu- Ala-Asn-Arg-Val-Gly-NH2 |
| Compound 24 | eicosanedioic acid-γGlu-Gly-Gly-Gly-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Arg-Val-Gly-NH2 |
| Compound 25 | eicosanedioic acid-γGlu-Gly-Gly-Gly-Gly-Gly-Thr-Asn-Leu- Ser-Val-Pro-Asn-Pro-Leu-Gly-Phe-Phe-Pro-Asp-His- Gln-Leu-Asp-Pro-Ala-Phe-Gly-Ala-Asn-Ser-Asn-Asn- Pro-Asp-Trp-Asp-Phe-Asn-Pro-Asn-Lys-Asp-His-Trp- Pro-Glu-Ala-Asn-Arg-Val-Gly-NH2 |
| Compound 26 | eicosanedioic acid-γGlu-Gly-Gly-Gly-Gly-Gly-Gly-Thr-Asn- Leu-Ser-Val-Pro-Asn-Pro-Leu-Gly-Phe-Phe-Pro-Asp- His-Gln-Leu-Asp-Pro-Ala-Phe-Gly-Ala-Asn-Ser-Asn- Asn-Pro-Asp-Trp-Asp-Phe-Asn-Pro-Asn-Lys-Asp-His- Trp-Pro-Glu-Ala-Asn-Arg-Val-Gly-NH2 |
| Compound 27 | eicosanedioic acid-γGlu-Gly-Gly-Gly-Gly-Gly-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- Asn-Asn-Pro-Asp-Trp-Asp-Phe-Asn-Pro-Asn-Lys-Asp- |
| | His-Trp-Pro-Glu-Ala-Asn-Arg-Val-Gly-NH2 |
| Compound 28 | eicosanedioic acid-γGlu-Gly-Gly-Ser-Gly-Ser-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- Asn-Asn-Pro-Asp-Trp-Asp-Phe-Asn-Pro-Asn-Lys-Asp- His-Trp-Pro-Glu-Ala-Asn-Arg-Val-Gly-NH2 |
| Compound 29 | eicosanedioic acid-γGlu-Gly-Thr-Asn-Leu-Ser-Val-Pro-Asn- Pro-Leu-Gly-Phe-Phe-Pro-Asp-His-Gln-Leu-Asp-Pro- Ala-Phe-Gly-Ala-Asn-Ser-Asn-Asn-Pro-Asp-Trp-Asp- Phe-Asn-Pro-Asn-Lys-Asp-His-Trp-Pro-Glu-Ala-Asn- Gln-Val-Gly-NH2 |
| Compound 30 | eicosanedioic acid-γGlu-PEG₁CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH₂ |
| Compound 31 | eicosanedioic acid-γGlu-PEG₂CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH₂ |
| Compound 32 | eicosanedioic acid-γGlu-PEG₃CH₂CO-Gly-Thr-Asn-Leu-Ser- Val-Pro-Asn-Pro-Leu-Gly-Phe-Phe-Pro-Asp-His-Gln- |
| | Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH2 |
| Compound 33 | eicosanedioic acid-γGlu-PEG₄CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH₂ |
| Compound 34 | eicosanedioic acid-γGlu-PEG5CH2CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH2 |
| Compound 35 | eicosanedioic acid-γGlu-PEG₆CH₂CO-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH₂ |
| Compound 36 | eicosanedioic acid-γGlu-Gly-Gly-Thr-Asn-Leu-Ser-Val-Pro- Asn-Pro-Leu-Gly-Phe-Phe-Pro-Asp-His-Gln-Leu-Asp- Pro-Ala-Phe-Gly-Ala-Asn-Ser-Asn-Asn-Pro-Asp-Trp- Asp-Phe-Asn-Pro-Asn-Lys-Asp-His-Trp-Pro-Glu-Ala- Asn-Gln-Val-Gly-NH₂ |
| Compound 37 | eicosanedioic acid-γGlu-Gly-Gly-Gly-Thr-Asn-Leu-Ser-Val- Pro-Asn-Pro-Leu-Gly-Phe-Phe-Pro-Asp-His-Gln-Leu- Asp-Pro-Ala-Phe-Gly-Ala-Asn-Ser-Asn-Asn-Pro-Asp- Trp-Asp-Phe-Asn-Pro-Asn-Lys-Asp-His-Trp-Pro-Glu- Ala-Asn-Gln-Val-Gly-NH2 |
| Compound 38 | eicosanedioic acid-γGlu-Gly-Gly-Gly-Gly-Thr-Asn-Leu-Ser- Val-Pro-Asn-Pro-Leu-Gly-Phe-Phe-Pro-Asp-His-Gln- Leu-Asp-Pro-Ala-Phe-Gly-Ala-Asn-Ser-Asn-Asn-Pro- Asp-Trp-Asp-Phe-Asn-Pro-Asn-Lys-Asp-His-Trp-Pro- Glu-Ala-Asn-Gln-Val-Gly-NH2 |
| Compound 39 | eicosanedioic acid-γGlu-Gly-Gly-Gly-Gly-Gly-Thr-Asn-Leu- Ser-Val-Pro-Asn-Pro-Leu-Gly-Phe-Phe-Pro-Asp-His- Gln-Leu-Asp-Pro-Ala-Phe-Gly-Ala-Asn-Ser-Asn-Asn- Pro-Asp-Trp-Asp-Phe-Asn-Pro-Asn-Lys-Asp-His-Trp- Pro-Glu-Ala-Asn-Gln-Val-Gly-NH2 |
| Compound 40 | eicosanedioic acid-γGlu-Gly-Gly-Gly-Gly-Gly-Gly-Thr-Asn- Leu-Ser-Val-Pro-Asn-Pro-Leu-Gly-Phe-Phe-Pro-Asp- His-Gln-Leu-Asp-Pro-Ala-Phe-Gly-Ala-Asn-Ser-Asn- Asn-Pro-Asp-Trp-Asp-Phe-Asn-Pro-Asn-Lys-Asp-His- Trp-Pro-Glu-Ala-Asn-Gln-Val-Gly-NH2 |
| Compound 41 | eicosanedioic acid-γGlu-Gly-Gly-Gly-Gly-Gly-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- |
| | Asn-Asn-Pro-Asp-Trp-Asp-Phe-Asn-Pro-Asn-Lys-Asp- His-Trp-Pro-Glu-Ala-Asn-Gln-Val-Gly-NH2 |
| Compound 42 | eicosanedioic acid-γGlu-Gly-Gly-Ser-Gly-Ser-Gly-Gly-Thr- Asn-Leu-Ser-Val-Pro-Asn-Pro-Leu-Gly-Phe-Phe-Pro- Asp-His-Gln-Leu-Asp-Pro-Ala-Phe-Gly-Ala-Asn-Ser- Asn-Asn-Pro-Asp-Trp-Asp-Phe-Asn-Pro-Asn-Lys-Asp- His-Trp-Pro-Glu-Ala-Asn-Gln-Val-Gly-NH₂ |

### Example 2 Determination of the effect of inhibition of HBV infection

The experimental groups included blank group, solvent group, positive control and compounds to be tested. The test concentration was 0.5 nM, and the positive drug was Myrcludex B. The HBeAg/HBsAg in the serum was detected every 3 days after infection.

### 1. Virus concentration

(1) The hepAD38 cells were cultured and the culture supernatant was collected. The collected culture supernatant from hepAD38 cells was centrifuged at 4°C and 4000 rpm/min for 30 min to remove cellular debris.
(2) 6×PEG8000 (Poly(ethylene glycol), sigma, SLBZ3934) solution was diluted to 1×PEG8000 by adding the centrifuged viral supernatant, and the mixture was placed on a horizontal shaker at 20rpm/min and 4°C overnight.
(3) On the next day, the virus concentrate was centrifuged at 4°C and 7000g for 30min and the supernatant was discarded. The precipitate was resuspended with serum-free and double antibiotic-free DMEM medium, and the resuspended virus concentrate solution was stored at 4°C.
(4) The multiplicity of infection (MOI) of virus concentrate solution was calculated as follows: 20µL of virus concentrate solution was taken, diluted with 180µL of PBS and digested with DNase (to reduce the effect of residual plasmid). Then, column extraction was performed using Viral DNA/RNA Kit, and the HBV DNA was quantified by qPCR detection with primer of S region using the standard product, to calculate the multiplicity of infection, i.e. the ratio of infected virus to the number of cells.

### 2 Infection and detection assay

(1) HepG2-NTCP cells were cultured with 10% FBS DMEM medium containing 4µg/mL Doxycycline for 4 days to induce NTCP expression.
(2) On the night before the fourth day of culture, HepG2-NTCP cells were plated to 48-well culture dish coated with type I rat tail collagen (200 µL of collagen solution) at 4*10^4 cells per well.
(3) On the second day, the medium was replaced with HCM (HBMTM, LONZA, CC-3199\ singleQuots, LONZA, CC-4182) to induce blockage on proliferation of HepG2-NTCP cells. In addition, Doxycycline was added, along with the drug to be tested.
(4) After 24h of incubation with HCM, HCM was discarded. The concentrated virus solution was added to the petri dish at 1600 MOI, replenished to 150 µL with HCM, added with the drug to be tested, and then added with 150 µL of 8% PEG8000, 2% DMSO, and 4µg/mL Doxycycline. The mixture was gently mixed well, and then placed in an incubator at 37°C for infection overnight.
(5) After 16 h of infection, the supernatant was discarded. The cells were washed 5 times with PBS, and the medium was replaced with 300 µL of DMEM (containing 10% FBS, 2% DMSO, and 4 µg/mL Doxycycline) for incubation.
(6) Subsequently, the medium was changed every three days. 300µl of cell culture supernatant was collected at the corresponding time point, and centrifuged at 3000g for 3min to remove the cell precipitate. The HBsAg and HBeAg in the supernatant were detected by using the fully automatic chemiluminescence immunoassay system, MAGLUMI X3 (New Industry Bio).

### 2 Experimental results

The experimental results are shown in the following table.

| HBeAg (IU/ml) | | | | | | |
|---|---|---|---|---|---|---|
| Infection time | 3 days | Inhibition rate % | 6 days | Inhibition rate % | 9 days | Inhibition rate % |
| Concentration of drug | 0.5nM | | 0.5nM | | 0.5nM | |
| Compound 6 | 0.95 | 58.15 | 4.39 | 54.08 | 5.93 | 41.58 |
| Compound 20 | 0.46 | 79.74 | 1.78 | 81.38 | 2.06 | 79.70 |
| Compound 34 | 0.96 | 57.71 | 4.28 | 55.23 | 6.00 | 40.89 |
| Myrcludex B | 0.75 | 66.96 | 3.1 | 67.57 | 4.16 | 59.01 |
| NC | 2.27 | | 9.56 | | 10.15 | |

| HBsAg (IU/ml) | | | | | | |
|---|---|---|---|---|---|---|
| Infection time | 3 days | Inhibition rate % | 6 days | Inhibition rate % | 9 days | Inhibition rate % |
| Concentration of drug | 0.5nM | | 0.5nM | | 0.5nM | |
| Compound 6 | 0.63 | 42.73 | 1.85 | 57.18 | 3.38 | 53.51 |
| Compound 20 | 0.41 | 62.73 | 0.57 | 86.81 | 0.61 | 91.61 |
| Compound 34 | 0.67 | 39.09 | 1.83 | 57.64 | 3.23 | 55.57 |
| Myrcludex B | 0.48 | 56.36 | 1.02 | 76.39 | 1.87 | 74.28 |
| NC | 1.10 | | 4.32 | | 7.27 | |

### Example 3 Preliminary determination of pharmacokinetic properties

The test animals were cynomolgus monkeys, two male cynomolgus monkeys in each compound group, and the drug was administered subcutaneously at 0.1 mg/kg. The blood was collected from vein before the administration (0 h), 1h, 2h, 3h, 4h, 8h, 12h, 18h, 24h, 48h, 96h, 144h, and 168h after the administration, and then centrifuged to isolate plasma samples. The serum drug concentration of the corresponding compounds was determined in the plasma samples by liquid chromatography-mass spectrometry, and the half-life of the compound after subcutaneous (SC) administration is shown in the table below.

| **Compound** | **t_{1/2} (h)** |
|---|---|
| Compound 6 | 90.6 |

## Claims

1. A compound represented by Formula I: 
R-Gly-Thr-Asn-Leu-Ser-Val-Pro-Asn-Pro-Leu-Gly-Phe-Phe-Pro-Asp-His-Gln-Leu-Asp-Pro
-Ala-Phe-Gly-Ala-Asn-Ser-Asn-Asn-Pro-Asp-Trp-Asp-Phe-Asn-Pro-Asn-Lys-Asp-His-Trp-Pro-
Glu-Ala-Asn-AA1-Val-Gly-AA2
Formula I
wherein AA1 of Formula I is selected from the group consisting of Arg, Lys, Gln, Glu, and Cit;
AA2 of Formula I is NH₂ or OH; and
R of Formula I is HO₂C(CH₂)ₙ₁CO-(AA3)ₙ₂-(PEGₙ₃(CH₂)ₙ₄CO)ₙ₅-, or HO₂C(CH₂)ₙ₁CO-(AA3)ₙ₂-(AA4)ₙ₆-;
wherein, n1 is an integer selected from 10 to 20,
n2 is an integer selected from 1 to 5,
n3 is an integer selected from 1 to 30,
n4 is an integer selected from 1 to 5,
n5 is 0, or is an integer selected from 1 to 5, and
n6 is 0, or is an integer selected from 1 to 10;
AA3 is selected from the group consisting of yGlu, εLys, β-Ala, γ-aminobutyric acid, and 5-Ava; and
AA4 is selected from the group consisting of Ala, Gly, Leu, Ser, Thr, Tyr, Gln, Lys, Dao, Dah, Orn, Dab, Dap, Glu, Asp, Ada, Apm, and Asu.

2. The compound according to claim 1, wherein AA1 is selected from the group consisting of Arg, Lys, and Gln.

3. The compound according to claim 1 or 2, wherein AA2 is NH₂.

4. The compound according to any one of claims 1 to 3, wherein R is eicosanedioic acid-γGlu-(AA4)ₙ₆-, AA4 is selected from the group consisting of Gly, Ser, Thr, Asn, Leu, Val, and Pro; and n6 is 0 or an integer selected from 1 to 7.

5. The compound according to any one of claims 1 to 3, wherein R is eicosanedioic acid-γGlu-PEGₙ₃-CH₂CO- and n3 is an integer selected from 1 to 6.

6. The compound according to any one of claims 1 to 5, wherein the compound is in a form selected from the group consisting of a pharmaceutically acceptable salt thereof, a solvate thereof, a chelate thereof, a non-covalent complex thereof, a prodrug thereof, and a mixture thereof.

7. Use of the compound according to any one of claims 1 to 6 in the manufacture of a long-acting inhibitor against entry of a hepatitis virus.

8. The use according to claim 7, wherein the hepatitis virus is hepatitis B virus or hepatitis D virus.

9. Use of the compound according to any one of claims 1 to 6 in the manufacture of a medicament for treating a chronic disease caused by hepatitis B and/or hepatitis D.

10. A drug for use in the treatment of a chronic disease caused by hepatitis B and/or hepatitis D, comprising the compound according to any one of claims 1 to 6.
